# EUROPEAN PATENT SPECIFICATION

(11) **EP 0 858 794 B1**
(45) Date of publication and mention of the grant of the patent: **05.08.2009**
(21) Application number: 98102432.6
(22) Date of filing: 12.02.1998
(51) Int. Cl.: A61Q 5/00, A61K 8/365

(54) **Hair care products**
Haarpflegemittel
Composition de soins des cheveux

(30) Priority: 12.02.1997 JP 2761997
(43) Date of publication of application: 19.08.1998
(73) Proprietor: KAO CORPORATION, Chuo-ku, Tokyo (JP)
(72) Inventor: Sakamoto, Yuichi, Sumida-ku, Tokyo (JP); Tanihara, Mamoru, Sumida-ku, Tokyo (JP)
(74) Representative: Kindler, Matthias

(56) References cited:
- EP-A- 0 673 647
- WO-A-95/13790
- DE-A- 3 029 263

## Description

### FIELD OF THE INVENTION

This invention relates to hair care products. More particularly, it relates to hair care products capable of imparting gloss and voluminousness to the hair, improving the combing properties thereof and, in particular, making the hair dried by damages glossy and soft.

### BACKGROUND OF THE INVENTION

In recent years, there have been employed various hair conditioning compositions for conditioning the hair, namely, imparting gloss, voluminousness and softness thereto and improving the combing properties thereof.

These hair conditioning compositions are classified into those which remain the hair conditioning components on the hair and those which are to be rinsed away after application.

Recently, α-hydroxy acids having a hydroxyl group at the α-position have attracted attention and the conditioning effects of these compounds are disclosed in EP No. 662316A and German Utility Model No. 29502426.

Further, EP No. 673647A discloses dermatological compositions which comprise a salt of lactic acid containing 70 to 100 % of L-lactic acid.

Furthermore, EP No. 684040A discloses skin treatment compositions comprising α-hydroxy acids, which contain at least 60 % of L-compounds, with N-acetyl-L-cysteine.

However, each of these compositions is insufficient in the conditioning effects, in particular, when applied to the hair having dry feel by damages.

WO 95/13790 A discloses non-irritating cosmetic compositions comprising α-hydroxy carboxylic acids such as L-lactic acid. The cosmetic composition is used for improving the skin condition.

DE 30 29 263 A discloses the use of whey serum in cosmetic compositions such as a shampoo. Acidic whey serum contains inter alia L(+)-lactic acid, D,L-lactic acid and other components. The cosmetic composition may be optionally enriched with L-lactic acid.

EP-A-0 727 204 describes hair washing agents for improving the combing properties, and imparting volume and gloss to hair. The washing agent contains 0.1 wt.% lactic acid, 0.05 wt.% citric acid, and pyrrolidone carboxylate.

### SUMMARY OF THE INVENTION

Accordingly, an object of the present invention is to provide hair care products which impart gloss, voluminousness and softness to the hair and improve the combing properties thereof.

Under these circumstances, the present inventors have found out that a specific lactic acid, i.e., L-lactic acid has excellent conditioning effects which are never achieved by other α-hydroxy acids including DL-lactic acid, thus completing the present invention.

Accordingly, the present invention provides a hair care product which comprises lactic acid or a salt thereof and an α-hydroxy acid other than lactic acid, wherein the ratio of L-lactic acid to the total lactic acid ranges from 60 to 100 % by weight, and wherein said α-hydroxy acid is at least one substance selected from the group consisting of citric acid, malic acid, glycolic acid and tartaric acid.

The lactic acid or salt thereof to be used in the present invention is one containing L-lactic acid at a higher ratio as compared with D-lactic acid which will be referred to as "lactic acid or salt thereof which is rich in the L-compound", hereinafter.

The ratio of L-lactic acid to the total lactic acid is from 60 to 100 % by weight, preferably from 85 to 100 % by weight. Although the pure L-lactic acid is preferred for the present invention, it tends to be racemized with the passage of time into an equilibrium mixture containing about 85 % by weight of L-lactic acid with about 15 % by weight of D-lactic acid.

Such a mixture is also useful for the present invention.

At present, lactic acid is commercially available in the form of aqueous solutions containing 10 to 90 % by weight of lactic acid in the form of the D- and L-compounds or a racemic modification. It is convenient in the present invention to use these solutions, so long as they contain L-lactic acid at a higher ratio as compared with D-lactic acid. Also, it is known that L-lactic acid can be obtained by extracting fermentation products of specific plants. Aqueous solutions of the L-lactic acid obtained by the fermentation of plants sometimes contain other α-hydroxy acids.

Mixture of the other α-hydroxy acids can be also used so long as the effect of the present invention is not worsened thereby.

Examples of the α-hydroxy acids other than L-lactic acid which are used in the present invention include citric acid, malic acid, glycolic acid and tartaric acid.

The ratio of L-lactic acid to the total lactic acid can be determined by measuring the specific rotation of the L-lactic acid in the conventional manner. The content of L-lactic acid of 60 % by weight corresponds to an optical purity of the L-compound of 20 % ee.

The salts of the lactic acid which can be used in the present invention are not particularly restricted, so long as they are inorganic and/or organic salts formed by reacting the lactic acid which is rich in the L-compound with various inorganic and/or organic bases. Examples of the inorganic bases include hydroxides, oxides, carbonates and hdyrogencarbonates of alkali metals and alkaline earth metals such as sodium, potassium, lithium, calcium and magnesium. Also, ammonium hydroxide and trialkylammonium hydroxides are useful as the inorganic bases. Examples of the organic bases include nitrogen-containing bases such as primary, secondary and tertiary amines; compounds having imino, guanidino, imidazolino, imidazolyl or pyridyl group; amino acids, peptides and proteins.

Examples of the amines as described above include those having alkyl and/or aryl groups. It is preferable to use therefor mono-, di- or trialkylamines such as propylamine, dipropylamine and triethylamine; arylamines such as aniline, methylaniline and propylanilihe; monoalkanolamines, dialkanolamines, trialkanolamines and alkylalkanolamines. The amino acids are typified by glycine, alanine, valine, leucine, isoleucine, serine, threonine, cysteine, cystine, tryptophan, ornithine, carnosine (alanylhistidine), 4-aminobutanoic acid and citrulline (α-amino-α-ureidovaleric acid). Among these, the most desirable ones are basic amino acids such as lysine, histidine and arginine. Moreover, it is also possible to use, as the base, lecithin, phosphatidylethanolamine, phosphatidylserine or sphingomyelin.

Although the content of the lactic acid or salt thereof which is rich in the L-compound in the hair care products of the present invention may be 0.005 % by weight or more, it is preferable to vary the content thereof depending on the type of the hair care products. When the hair care products are those which are used without washing away after application (e.g., hair blow, hair spray, hair foam, styling lotion, hair gel, and hair mist), the desired effects can be achieved by using the lactic acid or salt thereof which is rich in the L-compound in a relatively small amount. In these cases, excellent conditioning effects can be established by using the lactic acid or salt therteof which is rich in the L-compound in an amount of preferably at least 0.005 % by weight, particularly preferably from 0.02 to 20 % by weight, based on the total weight of the hair care product.

When the hair care products are those which are to be washed away after application (e.g., shampoo, conditioner, and treatment), excellent conditioning effects can be established by using the lactic acid or salt thereof which is rich in the L-compound in an amount of preferably at least 0.02 % by weight, particularly preferably from 0.05 to 20 % by weight, based on the total weight of the hair care product.

In the case of the hair care products to be washed away after application, the penetration of the lactic acid which is rich in the L-compound into the hair can be accelerated and the conditioning effects can be thus improved by cleansing the hair surface. Accordingly, the effects of the lactic acid which is rich in the L-compound can be enhanced by using anionic surfactants, ampholytic surfactants or nonionic surfactants together therewith for cleansing the hair surface.

Either one of these surfactants or a mixture of two or more thereof may be used in the present invention. The content of these surfactants based on the total weight of the hair care product preferably ranges from 3 to 60 % by weight, more preferably from 5 to 30 % by weight.

By further adding cationic surfactant(s), the adsorption of the lactic acid which is rich in the L-compound onto the hair can be promoted and thus the conditioning effects can be further improved.

Examples of the cationic surfactants are as follows.
(1) Quaternary ammonium salts of the ring-opened imidazoline type having alkyl or alkenyl group(s) having 7 to 21 carbon atoms.
(2) Mono-long chain alkyl quaternary ammonium salts having a linear or branched alkyl or alkenyl group having 8 to 24 carbon atoms which may be linked by oxygen atom(s) or acid amido group(s), or which may be substituted on a hydroxyl group.
(3) Di-long chain alkyl quaternary ammonium salts having linear or branched alkyl or alkenyl groups having 8 to 24 carbon atoms which may be linked by oxygen atom(s) or acid amido group(s), or which may be substituted on a hydroxyl group.
(4) Quaternary ammonium salts having asymmetric di-long chain alkyl or alkenyl groups having, in a single molecule, a branched alkyl group having 8 to 28 carbon atoms which may be linked by oxygen atom(s) or acid amido group(s), or which may be substituted on a hydroxyl group and a linear alkyl or alkenyl group having 8 to 22 carbon atoms which may be linked by oxygen atom(s) or acid amido group(s), or which may be substituted on a hydroxyl group.

The content of these cationic surfactants based on the total weight of the hair care product preferably ranges from 0.1 to 10 % by weight, more preferably from 0.5 to 5 % by weight.

The hair care products of the present invention may further contain conditioning agents such as cationic polymers, ampholytic polymers, silicones and silicone derivatives. By using these components, a film is formed on the hair surface. Thus, the adsorption of the lactic acid which is rich in the L-compound onto the hair can be promoted and further improved conditioning effects can be exerted.

Examples of the cationized polymers which can be used as the conditioning agents in the present invention include cationized cellulose derivatives, cationic starch, cationized guar gum derivatives, diallyl quaternary ammonium homopolymers, diallyl quaternary ammonium salt/acrylamide copolymers, diallyl quaternary ammonium salt/acrylic acid copolymers, diallyl quaternary ammonium salt/acrylic acid/acrylamide terpolymers, quaternized polyvinylpyrrolidone derivatives, polyglycol/polyamine condensates, etc. Among these components, it is preferable to use cationized cellulose derivatives, diallyl quaternary ammonium homopolymers, and diallyl quaternary ammonium salt/acrylamide and/or acrylic acid copolymers.

Among the silicones and silicone derivatives which can be used as the conditioning agents described above, it is particularly preferable to use dimethylpolysiloxane, methylphenylpolysiloxane, polyether-modified silicones, amino-modified silicones, etc. Either one of these components or a mixture of two or more thereof may be employed in the hair care products of the present invention.

The content of the conditioning agents which can be used in the present invention is preferably 0.01 % by weight or more, particularly preferably from 0.1 to 20 % by weight, based on the total weight of the hair care product.

The hair care products of the present invention may further contain various additives, so long as they exert no undesirable effect (e.g., decomposition) to L-lactic acid. For example, the following components which have been ordinarily employed in hair care products may be added thereto, if needed, so long as the effects of the present invention are not worsened thereby: water-soluble polymers such as methyl cellulose, hydroxyethyl cellulose, carboxyvinyl polymer and polysaccharides such as xanthan gum; viscosity controlling agents such as polyoxyalkylene sorbitan esters, polyoxyethylene glycol distearate and ethanol; humectants such as sorbitol and glycerol; chelating agents such as ethylenediaminetetraacetic acid (EDTA) and phosphonates; preservatives such as methylparaben and butylparaben; nutritional components such as vitamins and precursors thereof, animal or vegetable extracts such as lecithin and gelatin and derivatives thereof; fine powders of polymers such as nylon and polyethylene; anti-inflammatory agents such as potassium glycyrrhizinate; bactericides and anti-dandruff agents such as triclosan, triclocarban, octopirox and zinc pyrithione; antioxidants such as dibutylhydroxytoluene; pearling agents, UV absorbers, pH regulating agents, colorants and perfumes.

The hair care products of the present invention can be produced by conventional methods, for example, by mixing the above-mentioned components with stirring at room temperature or under heating to thereby obtain emulsions, aqueous solutions, gels, pastes, etc.

The pH value of the hair care products of the present invention generally ranges from 2 to 9, preferably from 3.5 to 7.5.

The hair care products of the present invention may be in the form of those which are to be washed away after application (e.g., shampoo, rinse, rinse-in-shampoo, hair treatment, and hair conditioner) or those which are used without washing away after application (e.g., hair blow, hair spray, hair foam, styling lotion, hair gel, and hair mist).

### EFFECT OF THE INVENTION

The hair care products of the present invention can impart gloss and voluminousness to the hair and improve the combing properties thereof. When applied to the hair dried by damages, in particular, they can impart gloss and softness thereto.

### EXAMPLES

To further illustrate the present invention in greater detail, and not by way of limitation, the following Examples will be given.

### Example 1

Shampoo samples having the compositions as shown in Table 1 (Inventive products 3, 4 and Comparative products 1 1*, 2*, 5*, 6*, 3) were produced by the conventional method. After using each sahmpoo samples, the hair was evaluated in slipperiness, softness and gloss. Moreover, the softness and gloss of damaged hair treated thereby were evaluated. The results are shown in Table 1.

### (Evaluation method)

### (1) Texture of hair

2 g of each shampoo samples was applied to a human hair tress (weight: about 30 g, length: about 25 cm). After shampooing and rinsing, the hair tress was sufficiently towel-dried and then dried with a dryer. Next, the slipperiness, softness and gloss of the hair were evaluated in accordance with the following criteria.
- E:: Excellent.
- G:: Good.
- M:: Moderate.
- B:: Bad.

### (2) Texture of damaged hair

2 g of each shampoo samples was applied to a human hair tress (weight: about 30 g, length: about 25 cm) which had been damaged by perming twice. After shampooing and rinsing, the hair tress was sufficiently towel-dried and then dried with a dryer. Next, the softness and gloss of the hair were evaluated in accordance with the above-mentioned criteria.

**Table 1**

| | Inventive product | | | | | | Comparative product | | |
|---|---|---|---|---|---|---|---|---|---|
| Component ( wt. %) | 1* | 2* | 3 | 4 | 5* | 6* | 1 | 2 | 3 |
| Sodium polyoxyethylene lauryl ether sulfate (EO=3) | 15 | 15 | 10 | 15 | 15 | - | 15 | 15 | 10 |
| Diethanolamide laurate | 2 | 2 | 2 | 2 | - | 2 | 2 | 2 | 2 |
| | | | | | | | | | |
| Hydroxyethyl cellulose hydroxypropyltrimethylammonium chloride ether ("Catinal", mfd. by Toho Chemical Industry) | 2 | - | 2 | - | 2 | 12 | 2 | - | 2 |
| | | | | | | | | | |
| 2-Alkyl-N-carboxymethyl-N-hydroxyethylimidazolinium-betaine ("Amphitol 20Y", mfd. by Kao Corp.) | - | - | 0.5 | 0.5 | - | 0.5 | - | - | 0.5 |
| | | | | | | | | | |
| High-polymeric methylpolysiloxane (2 x 10⁷ cs) | - | 1 | - | 1 | - | - | - | 1 | - |
| | | | | | | | | | |
| Dimethylpolysiloxane (200 cs) | - | 1 | - | 1 | - | - | - | 1 | - |
| | | | | | | | | | |
| Poly-chlorinated (dimethylallyl-ammonium chloride)/acrylamide copolymer ("Merquat/550", mfd. by Merck & Co. Int) | - | 0.5 | 0.5 | - | 0.5 | - | - | 0.5 | 0.5 |
| | | | | | | | | | |
| L-lactic acid^{*1} | 0.5 | 1 | 1 | 0.5 | 1 | 3 | - | - | - |
| | | | | | | | | | |
| DL-lactic acid^{*1} | - | - | - | - | 0.5 | - | - | 1 | 0.5 |
| | | | | | | | | | |
| Citric acid | - | - | 0.5 | 0.5 | - | - | 0.5 | - | 0.5 |
| | | | | | | | | | |
| pH-Regulating agent/Preservative/ Colorant/Perfume | sq. | sq. | sq. | sq. | sq. | sq. | sq. | sq. | sq. |
| | | | | | | | | | |
| Water | ba. | ba. | ba. | ba. | ba. | ba. | ba. | ba. | ba. |
| | | | | | | | | | |
| Slipperiness of hair | G | E | E | E | E | E | B | G | G |
| | | | | | | | | | |
| Softness of hair | E | E | E | E | G | E | B | M | M |
| | | | | | | | | | |
| Gloss of hair | E | E | E | E | G | E | B | M | M |
| | | | | | | | | | |
| Softness of damaged hair | G | G | E | E | G | G | B | B | B |
| | | | | | | | | | |
| Gloss of damaged hair | G | E | E | G | G | E | B | B | B |

| | | | | | | | | | |
|---|---|---|---|---|---|---|---|---|---|
| *1: Guaranteed reagent manufactured by Wako Pure Chemical Industries, Ltd. sq.: sufficient quantity ba.: balance * Comparative product | | | | | | | | | |

### Example 2

Rinse samples having the compositions as shown in Table 2 (Inventive products 9, 10 and Comparative products 4, 7*, 8*, 11*, 6) were produced by the conventional method. After using each rinse samples, the hair was evaluated in slipperiness, softness and gloss. Moreover, the softness and gloss of damaged hair treated thereby were evaluated. The results are shown in Table 2.

### (Evaluation method)

### (1) Texture of hair

A human hair tress (weight: about 30 g, length: about 25 cm) was shampooed with a marketed shampoo containing anionic surfactants as a main component. Next, 2 g of each rinse samples was applied uniformly onto the hair. Then the hair tress was washed, rinsed with running water for 30 seconds, sufficiently towel-dried and then dried with a dryer. Next, the slipperiness, softness and gloss of the hair were evaluated in accordance with the following criteria.
- E:: Excellent.
- G:: Good.
- M:: Moderate.
- B:: Bad.

### (2) Texture of damaged hair

2 g of the shampoo was applied to a human hair tress (weight: about 30 g, length: about 25 cm) which had been damaged by perming twice. After shampooing and rinsing, the hair tress was sufficiently towel-dried and then dried with a dryer. Next, the softness and gloss of the hair were evaluated in accordance with the above-mentioned criteria.

**Table 2**

| | Inventive product | | | | | Comparative product | | |
|---|---|---|---|---|---|---|---|---|
| Component (wt. %) | 7* | 8* | 9 | 10 | 11* | 4 | 5 | 6 |
| Stearyltrimethylammonium chloride | 1.5 | 1.5 | 1.5 | 1.5 | 1.5 | 1.5 | 1.5 | 1.5 |
| | | | | | | | | |
| Cetyl alcohol | 2 | 2 | 2 | 3 | 3 | 2 | 2 | 3 |
| | | | | | | | | |
| Propylene glycol | 3 | 3 | 3 | 3 | 3 | 3 | 3 | 3 |
| | | | | | | | | |
| Liquid paraffin | - | - | 1 | 1 | - | - | - | 1 |
| | | | | | | | | |
| High-polymeric methylpolysiloxane (2 x 10⁷ cs) | - | 1 | - | 1 | - | - | 1 | - |
| | | | | | | | | |
| Dimethylpolysiloxane (200 cs) | - | 1 | - | 1 | - | - | 1 | - |
| | | | | | | | | |
| Amodimethicone emulsion (SM8702C)^{*2} | - | 0.5 | 0.5 | - | 0.5 | - | 0.5 | 0.5 |
| | | | | | | | | |
| L-lactic acid^{*1} | 0.5 | 1 | 1 | 0.5 | 1 | - | - | - |
| | | | | | | | | |
| DL-lactic acid^{*1} | - | - | - | - | 0.5 | - | 1 | 0.5 |
| | | | | | | | | |
| Citric acid | - | - | 0.5 | 0.5 | - | 0.5 | - | 0.5 |
| | | | | | | | | |
| pH-Regulating agent/Preservative/ | | | | | | | | |
| Colorant/Perfume | sq. | sq. | sq. | sq. | sq. | sq. | sq. | sq. |
| | | | | | | | | |
| Water | ba. | ba. | ba. | ba. | ba. | ba. | ba. | ba. |
| Slipperiness of hair | G | E | E | E | E | B | G | G |
| | | | | | | | | |
| Softness of hair | E | E | E | E | G | M | G | M |
| | | | | | | | | |
| Gloss of hair | E | E | E | G | G | B | M | M |
| | | | | | | | | |
| Softness of damaged hair | G | G | E | E | G | B | B | B |
| | | | | | | | | |
| Gloss of damaged hair | G | E | E | G | G | B | B | B |

| | | | | | | | | |
|---|---|---|---|---|---|---|---|---|
| *1: Guaranteed reagent manufactured by Wako Pure Chemical Industries, Ltd. *2: Product of Toray-Dow Corning Silicone, Ltd. sq.: sufficient quantity ba.: balance *: Comparative product | | | | | | | | |

### Example 3

A hair treatment sample having the composition as shown in Table 3 (Inventive product 12*) was produced by the conventional method and evaluation was performed based on the same criteria as in Example 2. The results are shown in Table 3.

**Table 3**

| Component (wt. %) | Invention product 12* |
|---|---|
| 2-Dodecylhexadecyltrimethylammonium chloride | 1.5 |
| | |
| Stearyltrimethylammonium chloride | 1 |
| | |
| Dimethylpolysiloxane (500 cs) | 1 |
| | |
| Cetostearyl alcohol | 3 |
| | |
| Liquid paraffin | 1 |
| | |
| Hydroxyethyl cellulose (1 % aq. solution, viscosity: 8000 cp) | 0.5 |
| | |
| Polyoxyethylene (5) oleyl ether | 0.5 |
| | |
| L-lactic acid^{*1} | 1 |
| | |
| pH-Regulating agent/Preservative/ Colorant/Perfume | sufficient quantity |
| | |
| Water | balance |
| Slipperiness of hair | G |
| | |
| Softness of hair | E |
| | |
| Gloss of hair | E |
| | |
| Softness of damaged hair | E |
| | |
| Gloss of damaged hair | E |

| | |
|---|---|
| *1: Guaranteed reagent manufactured by Wako Pure Chemical Industries, Ltd. * not according to the invention | |

### Example 4

Hair blow samples having the compositions as shown in Table 4 (Inventive products 13 to 17 and Comparative products 7 to 9) were produced by the conventional method. After using each hair blow samples, the hair was evaluated in slipperiness, softness and gloss. Moreover, the softness and gloss of damaged hair treated thereby were evaluated. The results are shown in Table 4.

### (Evaluation method)

### (1) Texture of hair

0.5 g of each hair blow samples was sprayed uniformly onto a human hair tress (weight: about 30 g, length: about 25 cm). After sufficiently drying with a dryer, the slipperiness, softness and gloss of the hair were evaluated in accordance with the following criteria.
- E:: Excellent.
- G:: Good.
- M:: Moderate.
- B:: Bad.

### (2) Texture of damaged hair

0.5 g of each hair blow samples was sprayed uniformly onto a human hair tress (weight: about 30 g, length: about 25 cm) which had been damaged by perming twice. After sufficiently drying with a dryer, the softness and gloss of the hair were evaluated in accordance with the above-mentioned criteria.

**Table 4**

| | Inventive product | | | | | Comparative product | | |
|---|---|---|---|---|---|---|---|---|
| Component (wt. %) | 13* | 14* | 15 | 16 | 17* | 7 | 8 | 9 |
| High-polymeric methylpolysiloxane (2 x 10⁷ cs) | - | 1.5 | 1.5 | - | 1.5 | - | 1.5 | - |
| | | | | | | | | |
| Dimethylpolysiloxane (200 cs) | - | 2 | 2 | - | 3 | - | 2 | - |
| | | | | | | | | |
| Poly chlorinated (dimethylallylammonium chloride)/acrylamide copolymer ("Merquat 550", mfd. by Merck & Co. Int) | - | - | - | 0.5 | 3 | - | - | 0.5 |
| | | | | | | | | |
| Amodimethicone emulsion (SM8702C)^{*2} | - | - | 0.5 | - | 0.5 | - | 0.5 | 0.5 |
| | | | | | | | | |
| Propylene glycol | 1 | 1 | 1 | 1 | 1 | 1 | 1 | 1 |
| | | | | | | | | |
| Ethanol | 10 | 10 | 10 | 10 | 10 | 10 | 10 | 10 |
| | | | | | | | | |
| L-lactic acid^{*1} | 0.5 | 1 | 1 | 0.5 | 1 | - | - | - |
| | | | | | | | | |
| DL-lactic acid^{*1} | - | - | - | - | 0.5 | - | 1 | 0.5 |
| | | | | | | | | |
| Citric acid | - | - | 0.5 | 0.5 | - | 0.5 | - | 0.5 |
| | | | | | | | | |
| pH-Regulating agent/Preservative/ Colorant/Perfume | sq. | sq. | sq. | sq. | sq. | sq. | sq. | sq. |
| Water | ba. | ba. | ba. | ba. | ba. | ba. | ba. | ba. |
| Slipperiness of hair. | E | E | E | E | E | B | G | G |
| | | | | | | | | |
| Softness of hair | G | E | E | E | G | B | B | M |
| | | | | | | | | |
| Gloss of hair | E | E | E | G | G | B | M | M |
| | | | | | | | | |
| Softness of damaged hair | G | G | E | E | G | B | B | B |
| | | | | | | | | |
| Gloss of damaged hair | G | E | E | G | G | B | B | B |

| | | | | | | | | |
|---|---|---|---|---|---|---|---|---|
| *1: Guaranteed reagent manufactured by Wako Pure Chemical Industries, Ltd. *2: Product of Toray-Dow Corning Silicone, Ltd. sq.: sufficiently quantity ba.: balance * comparative product | | | | | | | | |

### Example 5

An aerosol spraying agent sample having the composition as shown in Table 5 (Inventive product 18*) was produced by the conventional method and evaluation was performed based on the same criteria as in Example 4. The results are shown in Table 5.

**Table 5**

| Component (wt. %) | Invention product 18* |
|---|---|
| High-polymeric methylpolysiloxane (2 x 10⁷ cs) | 1 |
| | |
| Dimethylpolysiloxane (200 cs) | 5 |
| | |
| Poly-chlorinated (dimethylallylammonium chloride)/acrylamide copolymer ("Merquat 550", mfd. by Merck & Co. Int) | 0.5 |
| | |
| Propylene glycol | 1 |
| | |
| Propellant (nitrogen gas) | 0.5 |
| | |
| Ethanol | 20 |
| | |
| L-lactic acid^{*1} | 1 |
| | |
| pH-Regulating agent/Preservative/ Colorant/Perfume | sufficient quantity |
| | |
| Water | balance |
| Slipperiness of hair | E |
| | |
| Softness of hair | E |
| | |
| Gloss of hair | E |
| | |
| Softness of damaged hair | E |
| | |
| Gloss of damaged hair | G |

| | |
|---|---|
| *1: Guaranteed reagent manufactured by Wako Pure Chemical Industries, Ltd. * not according to the invention | |

### Example 6

A foaming agent sample having the composition as shown in Table 6 (inventive product 19*) was produced by the conventional method and evaluation was performed based on the same criteria as in Example 4. The results are shown in Table 6.

**Table 6**

| Component (wt. %) | Invention product 19* |
|---|---|
| High-polymeric methylpolysiloxane (2 x 10⁷ cs) | 1 |
| | |
| Dimethylpolysiloxane (200 cs) | 5 |
| | |
| Polyoxyethylene lauryl ether (EO=10) | 0.5 |
| | |
| Propylene glycol | 1 |
| | |
| Propellant (LP gas) | 10 |
| | |
| L-lactic acid^{*1} | 1 |
| | |
| pH-Regulating agent/Preservative/ Colorant/Perfume | sufficient quantity |
| | |
| Water | balance |
| Slipperiness of hair | E |
| | |
| Softness of hair | E |
| | |
| Gloss of hair | E |
| | |
| Softness of damaged hair | G |
| | |
| Gloss of damaged hair | E |

| | |
|---|---|
| *1: Guaranteed reagent manufactured by Wako Pure Chemical Industries, Ltd. * not according to the invention | |

## Claims

1. A hair care product which comprises lactic acid or a salt thereof and an α-hydroxy acid other than lactic acid,
wherein the ratio of L-lactic acid to the total lactic acid ranges from 60 to 100 % by weight, and wherein said α-hydroxy acid is at least one substance selected from the group consisting of citric acid, malic acid, glycolic acid and tartaric acid.

2. The hair care product as claimed in claim 1, wherein the content of said lactic acid or salt thereof is at least 0.005 % by weight based on the total weight of the hair care product.

3. The hair care product as claimed in claim 1, wherein the content of said lactic acid or salt thereof is at least 0.02 % by weight based on the total weight of the hair care product.

4. The hair care product as claimed in claim 3, which contains at least one surfactant selected from the group consisting of anionic surfactants, nonionic surfactants and ampholytic surfactants in an amount of from 3 to 60 % by weight based on the total weight of the hair care product.

5. The hair care product as claimed in claim 3, which further contains cationic surfactants in an amount of from 0.1 to 10 % by weight based on the total weight of the hair care product.

6. The hair care product as claimed in any of claims 1 to 5, which further contains at least one conditioning agent selected from the group consisting of cationic polymers, ampholytic polymers, silicones and silicone derivatives in an amount of at least 0.01 % by weight based on the total weight of the hair care product.

7. The hair care product as claimed in any of claims 1 to 6, wherein said salt is at least one inorganic salt selected from the group consisting of alkali metal lactates, alkaline earth metal lactates and ammonium lactates and/or at least one amino acid salt.

8. The hair care product as claimed in any of claims 1 to 7, which has a pH value of 2 to 9.

## Patentansprüche

1. Haarpflegeprodukt, das Milchsäure oder ein Salz davon und eine von Milchsäure unterschiedliche α-Hydroxysäure umfasst, worin das Verhältnis von L-Milchsäure zu Gesamtmilchsäure 60 bis 100 Gew.% beträgt, und worin die α-Hydroxysäure mindestens eine Substanz ist, die ausgewählt ist aus Zitronensäure, Äpfelsäure, Glykolsäure und Weinsäure.

2. Haarpflegeprodukt gemäss Anspruch 1, worin der Gehalt an Milchsäure oder eines Salzes davon mindestens 0,005 Gew.% auf Basis des Gesamtgewichts des Haarpflegeprodukts ist.

3. Haarpflegeprodukt gemäss Anspruch 1, worin der Gehalt an Milchsäure oder eines Salzes davon mindestens 0,02 Gew.% auf Basis des Gesamtgewichts des Haarpflegeprodukts ist.

4. Haarpflegeprodukt gemäss Anspruch 3, das mindestens ein Tensid, ausgewählt aus anionischen Tensiden, nicht-ionischen Tensiden und ampholytischen Tensiden, in einer Menge von 3 bis 60 Gew.% auf Basis des Gesamtgewichts des Haarpflegeprodukts enthält.

5. Haarpflegeprodukt gemäss Anspruch 3, das weiterhin kationische Tenside in einer Menge von 0,1 bis 10 Gew.% auf Basis des Gesamtgewichts des Haarpflegeprodukts enthält.

6. Haarpflegeprodukt gemäss mindestens einem der Ansprüche 1 bis 5, das weiterhin mindestens ein Konditionierungsmittel, ausgewählt aus kationischen Polymeren, ampholytischen Polymeren, Siliconen und Siliconderivaten, in einer Menge von mindestens 0,01 Gew.% auf Basis des Gesamtgewichts des Haarpflegeprodukts enthält.

7. Haarpflegeprodukt gemäss mindestens einem der Ansprüche 1 bis 6, worin das Salz mindestens ein anorganisches Salz, das ausgewählt ist aus Alkalimetallactaten, Erdalkalimetallactaten und Ammoniumlactaten, und/oder mindestens ein Aminosäuresalz ist.

8. Haarpflegeprodukt gemäss mindestens einem der Ansprüche 1 bis 7, das einen pH-Wert von 2 bis 9 hat.

## Revendications

1. Produit de soin capillaire qui comprend de l'acide lactique ou un sel de ce dernier et un acide α-hydroxy autre que l'acide lactique, où le rapport de l'acide L-lactique à l'acide lactique total est de 60 à 100 % en poids et où ledit acide α-hydroxy est au moins une substance sélectionnée dans le groupe constitué de l'acide citrique, de l'acide malique, de l'acide glycolique et de l'acide tartrique.

2. Produit de soin capillaire selon la revendication 1, où la teneur en ledit acide lactique ou son sel est d'au moins 0,005 % en poids par rapport au poids total du produit de soin capillaire.

3. Produit de soin capillaire selon la revendication 1, où la teneur en ledit acide lactique ou son sel est d'au moins 0,02 % en poids par rapport au poids total du produit de soin capillaire.

4. Produit de soin capillaire selon la revendication 3, lequel contient au moins un tensioactif sélectionné dans le groupe constitué de tensioactifs anioniques, de tensioactifs non ioniques et de tensioactifs ampholytes en une quantité de 3 à 60 % en poids par rapport au poids total du produit de soin capillaire.

5. Produit de soin capillaire selon la revendication 3, lequel contient en outre des tensioactifs cationiques en une quantité de 0,1 à 10 % en poids par rapport au poids total du produit de soin capillaire.

6. Produit de soin capillaire selon l'une quelconque des revendications 1 à 5, lequel contient en outre au moins un agent de conditionnement sélectionné dans le groupe constitué de polymères cationiques, de polymères ampholytes, de silicones et de dérivés siliconés en une quantité d'au moins 0,01 % en poids par rapport au poids total du produit de soin capillaire.

7. Produit de soin capillaire selon l'une quelconque des revendications 1 à 6, où ledit sel est au moins un sel inorganique sélectionné dans le groupe constitué des lactates de métaux alcalins, des lactates de métaux alcalino-terreux et des lactates d'ammonium et/ou au moins un sel d'acide aminé.

8. Produit de soin capillaire selon l'une quelconque des revendications 1 à 7, lequel a un pH de 2 à 9.
